# EUROPÄISCHE PATENTANMELDUNG

(11) **EP 1 238 672 A1**
(43) Veröffentlichungstag der Anmeldung: **11.09.2002**
(21) Anmeldenummer: 02005482.1
(22) Anmeldetag: 09.03.2002
(51) Int. Cl.: A61K 35/78, A61P 31/12

(54) **Pharmazeutisches Kombinationspräparat enthaltend einen Extrakt aus Süssholzwurzel und Eichenrinde**

(30) Priorität: 09.03.2001 DE 10112215
(71) Anmelder: Schmolz, Manfred, Dr., 72810 Gomaringen (DE)
(72) Erfinder: Schmolz, Manfred, Dr., 72810 Gomaringen (DE)
(74) Vertreter: Patentanwälte , Ruff, Wilhelm, Beier, Dauster & Partner

(57) **Zusammenfassung**

Ein pharmazeutisches Kombinationspräparat wird bereitge- gestellt, welches einen Extrakt aus Süßholzwurzel (Glycyrrhiza glabra) und Eichenrinde (Quercus spec.) enthält. Dieses pharmazeutische Kombinationspräparat ist vorzugsweise zur Behandlung von viralen Erkrankungen vorgesehen.

## Beschreibung

Die Erfindung betrifft ein pharmazeutisches Kombinationspräparat, welches insbesondere zur Behandlung von viralen Erkrankungen geeignet ist.

Entzündungen im Lippen- und Rachenraum sind ein sehr verbreitetes Problem. Diese Entzündungen gehen häufig auf virale Infektionen zurück. Verursacher solcher Infektionen sind oft die sogenannten Herpes-Viren. Hierbei gibt es viele verschiedene Arten dieser Viren, wobei nur einige davon beim Menschen eine Krankheit auslösen.

Obwohl diese verschiedenen Herpes-Viren sich unterschiedlich verhalten, ist ihnen allen eine unangenehme Eigenschaft eigen: wenn sie einmal in den Körper eingedrungen sind und eine Erstinfektion verursacht haben, verlassen sie den Körper nicht mehr. Vielmehr ziehen sie sich an den Nervenfasern entlang in die Nervenknoten als ruhende "latente" Viren zurück. Unter bestimmten Umständen können dann diese Viren erneut aktiv werden und ein Krankheitsbild hervorrufen.

Ein besonders weit verbreitetes, unangenehmes Problem stellt eine Herpes-Infektion dar, die sich als Bläschen an den Lippen, dem sogenannten Herpes labialis, äußert. Dies ist die häufigste Form einer Herpes-Erkrankung. Die Infektion beruht auf dem Herpes-Simplex-Virentyp I. Etwa 30 bis etwa 70 % der Bevölkerung werden bereits im Kindesalter mit diesem Virus infiziert. Allerdings treten die Symptome nicht zwangsläufig bei den Infizierten auf. Die Viren setzen sich nach der Primärinfektion in den Nervenknoten fest und verursachen bei vielen Betroffenen von Zeit zu Zeit die sehr unangenehmen sogenannten Herpes-Bläschen, die vorwiegend an den Lippen auftreten. Diese sogenannten Rezidive erscheinen vornehmlich unter bestimmten Umständen, beispielsweise unter Stress, extremer Sonnenbestrahlung, Hautreizung oder anderen Belastungen. Zunächst macht sich hierbei ein Juckreiz und ein Spannungsgefühl bemerkbar. Sodann treten gruppierte Bläschen auf gerötetem Grund auf, die später zu Krusten eintrocknen und abheilen.

Herpes-Viren können jedoch auch ganz andere Krankheitsbilder verursachen. Beispielsweise wird die Kinderkrankheit Windpocken gleichfalls von Herpes-Viren verursacht. Die Viren verbleiben als latente Viren im Körper und können viele Jahre später wieder aktiv werden und eine sogenannte Gürtelrose, den Herpes zoster, hervorrufen. Weiterhin können sich Herpes-Infektionen auch im Auge manifestieren oder sich als Herpes der Geschlechtsorgane oder virale Hirnhautentzündung äußern.

Es gibt eine Fülle von Hausrezepten, die bei Herpes-Erkrankungen, insbesondere Herpes labialis, empfohlen werden. Im allgemeinen sind diese Rezepte jedoch wenig wirksam. Bis zu einem gewissen Umfang hat sich der Wirkstoff Aciclovir als wirksam bei der Behandlung von Herpes-Bläschen an den Lippen erwiesen. Eine Therapie mit Aciclovir ist jedoch in jedem Fall rein symptomatisch, so daß es immer wieder zum Ausbruch von Rezediven, also einer Reaktivierung der latenten Viren, kommt.

Die Erfindung stellt sich daher die Aufgabe, ein pharmazeutisches Präparat bereitzustellen, welches für die Therapie von Krankheiten einsetzbar ist, die beispielsweise auf einer viralen Infektion beruhen. Insbesondere stellt sich die Erfindung die Aufgabe, ein Präparat bereitzustellen, welches für die Therapie von Symptonen im Lippen- und Rachenraum geeignet ist, die auf viralen Infektionen und insbesondere auf Infektionen mit Herpes-Viren zurückzuführen sind. Diese Aufgabe wird gelöst durch ein pharmazeutisches Kombinationspräparat, wie es in Anspruch 1 beschrieben ist. Bevorzugte Ausführungsformen dieses Kombinationspräparates sind in den Ansprüchen 2 bis 11 dargestellt. Die Ansprüche 12 und 13 betreffen die Verwendung bestimmter pflanzlicher Produkte. Der Wortlaut sämtlicher Ansprüche wird hiermit durch Bezugnahme zum Inhalt der Beschreibung gemacht.

Das erfindungsgemäße pharmazeutische Kombinationspräparat enthält einen Extrakt aus Süßholzwurzel (Glycyrrhiza glabra) und Eichenrinde (Quercus spec.). Ein solches Präparat ist in besonderer Weise für die Behandlung von viralen Erkrankungen geeignet. Besonders vorteilhaft können hiermit Krankheiten behandelt werden, deren Symptome sich im Lippen- und/oder Rachenraum äußern. Dies können beispielsweise Herpes-Erkrankungen sein, insbesondere Herpes labialis-Erkrankungen. Doch auch eine Vielzahl anderer Erkrankungen wird durch das erfindungsgemäße Kombinationspräparat wirksam behandelt. Beispielsweise kann das Präparat für die sehr unterschiedlichen Erkrankungen eingesetzt werden, die durch die verschiedenen Herpes-Viren verursacht werden. Jedoch sind die mit dem erfindungsgemäßen Präparat behandelbaren Erkrankungen keineswegs auf Herpes-Infektionen beschränkt. So können auch Erkältungskrankheiten, die auf viralen Infektionen beruhen, sehr wirksam mit dem Kombinationspräparat therapiert werden.

Weiterhin ist das erfindungsgemäße Kombinationspräparat auch für die Behandlung von Erkrankungen geeignet, die auf bakteriellen Infektionen beruhen oder die allgemein mit Entzündungserscheinungen einhergehen. In besonderer Weise ist das Kombinationspräparat auch für die Behandlung von grippalen Infekten geeignet.

Ein Bestandteil des erfindungsgemäßen pharmazeutischen Kombinationspräparates ist ein Extrakt aus Süßholzwurzel. Als Süßholzwurzel kommen die getrockneten Wurzeln der Süßholzpflanze (Glycyrrhiza glabra) zum Einsatz. Diese Pflanze ist der Familie der Schmetterlingsblütler zuzuordnen und kommt vor allem wildwachsend als Staudenpflanze von Südeuropa bis Westsibirien, Südamerika, Vorder- und Mittelasien sowie Australien vor. Weiterhin wird Süßholz auch auf warmen sandigen Böden vor allem in Osteuropa angebaut. Die Planze ist holzig und kann bis zu 2 m hoch werden. Zur Ernte der Wurzel werden nach 3 bis 4 Jahren die bis zu 2 cm dicken Nebenwurzeln abgeschnitten und getrocknet. Die Ernte kann alle drei Jahre vom Spätherbst bis zum Frühjahr erfolgen. Die Droge hat einen kaum wahrnehmbaren Geruch und schmeckt außerordentlich süß.

Die Wurzel und auch der Saft der Süßholzpflanze finden schon lange in der Medizin Verwendung. Beispielsweise wird die getrocknete Wurzel als Teemischung eingesetzt. Ein konzentrierter Extrakt der Süßholzwurzel soll bei der Behandlung von Magengeschwüren hilfreich sein. Besondere Verbreitung hat der eingedickte Süßholzsaft als wichtiger Bestandteil von Lakritzwaren erlangt.

Ein weiterer Bestandteil des erfindungsgemäßen pharmazeutischen Kombinationspräparates ist ein Extrakt aus Eichenrinde. Als Eichenrinde kommt beispielsweise die getrocknete Rinde junger Stämme und Zweige der Stiel- oder Sommereiche (Quercus robur) und der Stein- oder Wintereiche (Quercus petraea) zum Einsatz. Die Eichenrinde ist vor allem aus ost- und südosteuropäischen Ländern lieferbar, wo sie angebaut wird. Die Ernte der Rinde wird in der Regel von März bis April, also vor der Entwicklung der Blätter, bei einem Alter von etwa 3 bis etwa 10 Jahren der Bäume vorgenommen. Vor allem aufgrund des hohen Gerbstoffgehaltes wird die Eichenrinde als medizinisch wirksame Droge seit langem eingesetzt. Die hierdurch bedingte adstringierende Wirkung macht sie als Badezusatz zur Linderung von entzündlichen Hauterkrankungen und als Mittel zur Behandlung von Durchfallerkrankungen geeignet.

Erfindungsgemäß wird ein Extrakt aus diesen beiden Naturprodukten für die Herstellung eines pharmazeutischen Kombinationspräparates eingesetzt. Dieses Kombinationspräparat zeigt sehr überraschende positive Wirkungen bei der Behandlung verschiedener Erkrankungen, insbesondere viraler Erkrankungen. Der beobachtbare synergistische Effekt beider Bestandteile beruht vermutlich auf einer gegenseitigen Unterstützung von virulogischen und immunologischen Effekten der verschiedenen Inhaltsstoffe dieser Drogen.

Bei einer viralen Infektion wechselwirken die Viren mit Oberflächenrezeptoren von Zellen, woraufhin die Viren in die Zellen eindringen können. Innerhalb der Zellen vermehren sich die Viren auf Kosten der Zellen und verlassen anschließend die Zelle, wobei die Zelle zerstört wird. Einer der Effekte des erfindungsgemäßen pharmazeutischen Kombinationspräparates ist es vermutlich, daß die Oberflächenrezeptoren geschädigt werden, so daß eine Anheftung der Viren verhindert und so das Eindringen der Viren in die Zelle nicht stattfinden kann. Bei dieser Schädigung der Oberflächenrezeptoren handelt es sich vermutlich um eine unspezifische Wechselwirkung verschiedener Inhaltsstoffe des erfindungsgemäßen Kombinationspräparates mit den Oberflächenproteinen, sodaß das erfindungsgemäße Präparat eine relativ breitgefächerte Wirksamkeit entfaltet. Weiterhin beeinflußt das erfindungsgemäße Präparat vermutlich auch Vermehrungsprozesse der Viren innerhalb der Zelle.

Darüber hinaus induziert das erfindungsgemäße Kombinationspräparat vermutlich eine Vielzahl immunologischer Abwehrmechanismen. Hierzu zählt beispielsweise die Aktivierung von antigenspezifischen T-Lymphozyten durch Inhaltsstoffe des erfindungsgemäßen Kombinationspräparates. Diese aktivierten T-Lymphozyten greifen direkt die virusinfizierten Zellen an. Weiterhin kann von diesen aktivierten T-Lymphozyten Interferon-Gamma freigesetzt werden. Dieses verstärkt zum einen wiederum die Aktivierung der T-Lymphozyten und aktiviert darüber hinaus auch die natürlichen Killer-Zellen. Auch diese Zellen greifen wiederum die virusinfizierten Zellen an. Zusätzlich kann das Interferon-Gamma direkt auf die Virusvermehrung innerhalb der infizierten Zellen hemmend wirken. Insgesamt wirkt das erfindungsgemäße Kombinationspräparat aktivierend auf das Immunsystem und setzt eine Vielzahl von Mechanismen in Gang, die die Virusvermehrung und die virusinfizierten Zellen bekämpfen.

Neben der akuten Bekämpfung der Viren wirkt das erfindungsgemäße Kombinationspräparat ebenfalls gegen eine erneute Aktivierung der latenten Viren. Diese besonders vorteilhafte Wirkung des erfindungsgemäßen Kombinationspräparates beruht vermutlich auch auf den sich gegenseitig unterstützenden antiviralen und immunologischen Effekten des Präparates. Diese synergistischen Effekte sind vermutlich die Ursache für die überraschenden therapeutischen Erfolge des erfindungsgemäßen Präparates.

Durch die sehr unterschiedlichen Angriffspunkte des Präparates, die sich gegenseitig fördern, wird weiterhin vorteilhafterweise die Ausbildung von Resistenzen der jeweiligen Erreger vermieden. Solche sich entwickelnden Resistenzen stellen insbesondere im Krankenhausalltag ein sehr großes Problem dar. Dies trifft vor allem für chemisch definierte Präparate zu, die anfangs sehr wirksam sein können, aber später überhaupt keine Wirkung mehr zeigen, da die entsprechenden Erreger infolge von Mutationen oder ähnlichem nicht mehr auf dieses Präparat ansprechen. Bei der Verwendung des erfindungsgemäßen Kombinationspräparates ist vorteilhafterweise die Entwicklung solcher Resistenzen aufgrund der vermutlich sehr unterschiedlichen Wirkmechanismen ausgeschlossen.

Der Extrakt aus Süßholzwurzel und Eichenrinde als Bestandteil des erfindungsgemäßen Kombinationspräparates wird vorteilhafterweise mit Hilfe von Auszugsmitteln gewonnen, die in der Herstellung pflanzlicher Extrakte üblich sind. Es werden hierbei auch beispielsweise Ölextrakte oder Presssäfte der pflanzlichen Drogen vor der Erfindung mit umfasst.

Besonders bevorzugt sind Auszugsmittel, die hydrophile und vor allem lipophile Bestandteile der pflanzlichen Drogen extrahieren.

Vorteilhafterweise wird der Auszug mit einem organischen Lösungsmittel, vorzugsweise mit einem lipophilen, insbesondere einem mit Wasser mischbaren Lösungsmittel durchgeführt. Besonders bevorzugt ist der Einsatz von Extraktionspaaren, beispielsweise von einem mit Wasser mischbaren Lösungsmittel und Wasser. Vorteilhafterweise werden pharmazeutisch unbedenkliche Lösungsmittel verwendet.

In einer besonders bevorzugten Ausführungsform der Erfindung wird als Auszugs- bzw. Extraktionsmittel ein Gemisch aus Wasser und einem Alkohol, insbesondere Ethanol eingesetzt. Hierbei kann das Auszugsmittel aus etwa 20 bis etwa 80 Vol.% Ethanol und etwa 20 bis etwa 80 Vol. % Wasser bestehen. Besonders vorteilhaft ist ein Auszugsmittel, welches aus etwa 40 bis etwa 70 Vol.% Ethanol und etwa 30 bis etwa 60 Vol.% Wasser besteht. Hierbei kann deionisiertes Wasser oder aber auch normales Leitungswasser verwendet werden.

Die Extraktion der pflanzlichen Drogen kann in einem weiten Temperaturbereich durchgeführt werden. Bevorzugt sind hierbei Temperaturen von etwa 10 bis etwa 80 °C. Besonders bevorzugt sind Temperaturen von etwa 15 bis etwa 30 °C, insbesondere etwa Raumtemperatur. Unter diesen Bedingungen findet eine ausreichende Extraktion der pflanzlichen Inhaltsstoffe unter sehr praktikablen Bedingungen statt. Es kann jedoch auch bevorzugt sein, die Extraktion bei höheren Temperaturen durchzuführen, beispielsweise bei etwa 40 bis etwa 60 °C, insbesondere bei etwa 45 bis etwa 55 °C. Dies hat den Vorteil, daß die Extraktion beschleunigt stattfindet, sodaß die Extraktionszeiten verkürzt werden können.

In einer bevorzugten Ausführungsform der Erfindung wird die Extraktion ruhend durchgeführt, d.h., daß während der Extraktion mit einem geeigneten Extraktionsmittel der Ansatz nicht bewegt wird. Es ist jedoch durchaus möglich und unter Umständen vorteilhaft, daß während der Extraktion der Ansatz bewegt, beispielsweise gerührt oder geschüttelt wird.
Die Extraktion wird vorteilhafterweise über mehrere Stunden hinweg durchgeführt. Bevorzugte Zeiten hierbei sind etwa 2 bis etwa 48 Stunden. Die Dauer der Extraktionszeit kann in einer gewissen Wechselwirkung mit der gewählten Extraktionstemperatur stehen. So kann es vorteilhaft sein, eine Extraktion bei niedrigeren Temperaturen über einen längeren Zeitraum hinweg durchzuführen. Beispielsweise kann eine Extraktion bei Raumtemperatur über 24 Stunden hinweg, und eine Extraktion bei einer Temperatur von 50 °C über 6 Stunden hinweg vorteilhaft sein. In der Regel werden jedoch auch bei kürzeren Extraktionszeiten ausreichende Mengen der Inhaltsstoffe der pflanzlichen Drogen extrahiert und auch längere Extraktionszeiten sind im allgemeinen nicht nachteilig.

Für den Extrakt aus Süßholzwurzel und Eichenrinde werden etwa 1 bis etwa 5 Teile Süßholzwurzel und etwa 1 bis etwa 3 Teile Eichenrinde verwendet. Diese Teile beziehen sich auf die Gewichte der pflanzlichen Drogen. In einer besonders bevorzugten Ausführungsform der Erfindung werden für den Extrakt etwa 2 Teile Süßholzwurzel und etwa 1 Teil Eichenrinde verwendet. Dieses Verhältnis der beiden Drogen hat sich in der Wirkung als besonders vorteilhaft erwiesen. Darüber hinaus hat dieses Verhältnis den Vorteil, daß der eher muffige und etwas unangenehme Charakter der Eichenrinde durch das sehr süße und angenehme Süßholz überdeckt wird. Da das erfindungsgemäße Kombinationspräparat im Bereich des Lippen- und/oder Rachenraums eingesetzt werden kann, ist diese vom Süßholz bestimmte Geschmacksnote für den Patienten im allgemeinen angenehmer.

Bei der Herstellung des Extraktes ist es möglich, daß die Süßholzwurzel und die Eichenrinde getrennt voneinander mit einem geeigneten Auszugsmittel extrahiert werden, und daß diese beiden Extrakte nachträglich vereinigt werden. Andererseits ist es auch möglich, daß beide Drogen in einem gemeinsamen Ansatz, also als Mischung, extrahiert werden. Dies ist besonders vorteilhaft, da durch die gemeinsame Extraktion gewährleistet wird, daß die verschiedenen Inhaltsstoffe der pflanzlichen Drogen in Lösung bleiben.

Für die Herstellung des erfindungsgemäßen Kombinationspräparates können die üblichen Aufbereitungsformen der Drogen eingesetzt werden. Vorteilhafterweise wird die Süßholzwurzel und die Eichenrinde in getrockneter und/oder geschnittener Form eingesetzt. Diese sogenannten Schnittdrogen werden bevorzugt in einer Größe von etwa Linsen oder Bohnen verwendet. Dies hat den Vorteil, daß die pflanzlichen Teile nach erfolgter Extraktion problemlos im Ansatz sedimentieren und daß das Extrakt einfach abgegossen oder grobfiltriert werden kann. Es ist jedoch auch möglich, daß die Drogen als Pulver, also als gemahlene Pflanzenteile, eingesetzt werden. Für die weitere Aufarbeitung nach der Extraktion ist in diesem Fall in der Regel eine Filtration notwendig.

Der nach der Extraktion gewonnene Auszug kann auf verschiedene Weise weiterbearbeitet werden. Beispielsweise kann der Auszug aufkonzentriert werden, um so eine höhere Wirkstoffkonzentration zu erreichen. Im allgemeinen ist die Wirkstoffkonzentration des Auszugs jedoch ausreichend, so daß in einer besonders bevorzugten Ausführungsform des erfindungsgemäßen Kombinationspräparates der Extrakt, so wie er nach der Extraktion vorliegt, als flüssiger Auszug am Patienten verwendet wird. Dies ist besonders vorteilhaft, da so die Herstellung des Präparates ausgesprochen einfach und kostengünstig ist. Bei Verwendung eines wässrig-alkoholischen, insbesondere wässrig-ethanolischen Auszugsmittels ist aufgrund des Alkoholgehalts im allgemeinen auch kein weiterer Zusatz von Konservierungsmittel notwendig. Der flüssige Auszug kann daher beispielsweise ohne weiteres als Tinktur auf die Lippen oder im Rachenraum aufgetragen werden.

Vorteilhafterweise ist das erfindungsgemäße Kombinationspräparat für eine solche topische, d.h. also örtliche Auftragung vorgesehen. Diese topische Auftragung ist besonders für die Behandlung von Herpes-Bläschen oder von lokalisierbaren Entzündungen im Rachenraum geeignet. Die Auftragung kann hierbei mit dem Finger oder mit anderen Hilfsmitteln vorgenommen werden. Besonders bevorzugt ist eine Auftragung mit Hilfe eines Applikators, da hierdurch Verunreinigungen des pharmazeutischen Präparates vermieden werden. Beispielsweise kann das Präparat mit einem Zellstofftupfer, insbesondere einem herkömmlichen Wattestäbchen, aufgetragen werden. Es sind jedoch auch andere Applikatoren durchaus möglich. Besonders vorteilhaft ist hierbei, wenn der Applikator ein gewisses Flüssigkeitsreservoir aufweist.

Das erfindungsgemäße Kombinationspräparat kann weiterhin durch Gurgeln oder Schlucken angewendet werden. Dies ist besonders bei Entzündungen im Rachenraum, beispielsweise bei grippalen Infekten, sinnvoll. So kann bei einem ersten Kratzen im Hals das erfindungsgemäße Kombinationspräparat, eventuell in einer geeigneten Verdünnung, gegurgelt werden, um so die Entzündung wirksam zu bekämpfen.

In einer weiteren bevorzugten Ausführungsform liegt das erfindungsgemäße pharmazeutische Kombinationspräparat mindestens teilweise in getrockneter Form vor. Hierfür wird das Extraktionsmittel nach der Extraktion durch Verdampfung, beispielsweise auch durch Gefriergetrocknung, verdampft, so daß die extrahierten Inhaltsstoffe der Drogen in getrockneter Form vorliegen. Diese Inhaltsstoffe können dann in verschiedener Weise weiterverwendet oder weiterverarbeitet werden. Beispielsweise kann dieses Pulver direkt als pharmazeutischen Präparat eingesetzt werden. Es kann beispielsweise mit Wasser oder einem anderen Lösungsmittel aufgelöst werden, um so eine Gurgel- oder Trinklösung bereitzustellen. Andererseits kann der getrocknete Extrakt auch weiter in einer Salbe oder in einer Paste verarbeitet werden. In dieser Form ist das Präparat auch für eine topische Auftragung sehr geeignet. Der Extrakt kann jedoch auch in andere herkömmliche pharmazeutische Darreichungsformen verarbeitet werden, beispielsweise in Zäpfchen oder Tabletten.

In einer weiteren bevorzugten Ausführungsform liegt das erfindungsgemäße Kombinationspräparat als Mischung mit einem als Nahrungsmittel, Genußmittel und/oder Körperpflegemittel dienenden Träger vor. So kann der Extrakt beispielsweise in ein Kaubonbon oder in ein Kaugummi oder auch in Zahnpasta eingearbeitet sein. So gelangen die wirksamen Inhaltsstoffe des erfindungsgemäßen Kombinationspräparates durch Kauen oder Lutschen bzw. durch Zähneputzen an den Ort der Entzündung und können hier ihre Wirkung entfalten. Die Einarbeitung in ein Kaugummi beispielsweise hat den Vorteil, daß ein solches Kaugummi über einen relativ langen Zeitraum im Mund- bzw. Rachenraum verbleibt und so die wirksamen Inhaltsstoffe des Präparates hier ihre Wirkung langfristig entfalten können.

Weiterhin wird von der Erfindung ein Verfahren zur Herstellung einer pharmazeutischen Zusammensetzung umfaßt, die dadurch gekennzeichnet ist, daß ein Extrakt aus Süßholzwurzel und Eichenrinde hergestellt wird. Bezüglich der Einzelheiten dieses Verfahrens wird auf das oben Gesagte bezug genommen.

Schließlich umfaßt die Erfindung die Verwendung von Süßholzwurzel und Eichenrinde zur Herstellung einer pharmazeutischen Zusammensetzung. Diese pharmazeutische Zusammensetzung dient bevorzugterweise zur Behandlung von viralen Erkrankungen. Weiterhin umfaßt die Erfindung die Verwendung von Süßholzwurzel und Eichenrinde zur Behandlung von viralen Erkrankungen. Bezüglich dieser Verwendungen wird auf das oben Gesagte verwiesen.

Weitere Merkmale und Vorteile der Erfindung ergeben sich aus den nachfolgend beschriebenen Beispielen in Verbindung mit den Unteransprüchen. Die sich hieraus ergebenden einzelnen Merkmale der Erfindung können jeweils für sich oder in Kombination miteinander verwirklicht sein.

### Beispiele

### Beispiel 1

Für die Herstellung eines Extraktes werden 4 g getrocknete und in kleine Stücke geschnittene Süßholzwurzel und 2 g getrocknete und in kleine Stücke geschnittene Eichenrinde gemischt. Diese Drogen werden in ein Becherglas gegeben und 100 ml Extraktionsmittel, welches aus 60 ml 96 %igen Ethanol und 40 ml deionisiertem Wasser besteht, hinzugegeben. Die Drogen bilden hierbei etwa ein Viertel bis ein Drittel Sediment im Bezug auf den gesamten Ansatz. Für die Extraktion wird der Ansatz über etwa 24 Stunden bei Raumtemperatur stehen gelassen. In einem anderen Ausführungsbeispiel wird die Extraktion über 6 Stunden bei etwa 50 °C durchgeführt. Anschließend wird der Überstand dekantiert und durch einen groben Filter gegeben. Der so gewonnene Extrakt wird direkt in geeignete Gefäße portioniert und abgefüllt und als pharmazeutisches Kombinationspräparat eingesetzt.

Dieses Präparat wurde von freiwilligen Probanden im Mund- und/oder Rachenraum angewendet. Bei sich ankündigenden oder schon aufgetretenen Herpes-Bläschen wurde das Präparat mit einem Wattestäbchen auf die betroffenen Hautstellen mehrfach am Tag aufgetragen. Bei anderen Entzündungen im Mund- und/oder Rachenraum wurde die Lösung ebenfalls entweder lokal aufgetragen oder es wurde mit einer Verdünnung dieser Lösung gegurgelt.

In allen Fällen zeigte sich eine deutliche Linderung der Beschwerden. In Bezug auf eine Herpes-Erkrankung im Lippenbereich war die Wirksamkeit des erfindungsgemäßen Kombinationspräparates zumindest vergleichbar mit dem herkömmlich verwendeten Aciclovir. Darüber hinaus zeigte das erfindungsgemäße Kombinationspräparat eine deutlich bessere Langzeitwirkung als Aciclovir. Nach Behandlung der akuten Herpes-Bläschen mit Aciclovir und Abheilung dieser Bläschen traten bei der herkömmlichen Behandlung bei einem Probanden sehr häufig innerhalb der folgenden 14 Tage erneut Rezidive auf. Durch die Behandlung mit dem erfindungsgemäßen Kombinationspräparat konnten zum einen die akuten Herpes-Beschwerden deutlich gebessert werden sowie auch diese erneuten Rezidive verhindert werden. Dieser langfristige Effekt, welcher vermutlich auf die synergistischen Wirkungen der verschiedenen Inhaltsstoffe des Kombinationspräparates auf das Immunsystem zurückzuführen ist, zeigt die deutlichen Vorteile des erfindungsgemäßen Kombinationspräparates gegenüber herkömmmlichen Präparaten bei der Behandlung von viralen Erkrankungen.

### Beispiel 2

Der folgende Vergleichsversuch zeigt, dass durch eine ethanolische Extraktion einer Mischung aus Eichenrinde und Süßholzwurzel ein anderes Inhaltsstoffspektrum erfasst wird als durch eine Extraktion mit reinem Wasser, wie sie beispielsweise in der japanischen Patentzusammenfassung JP 07179354 A beschrieben ist. Die in der japanischen Schrift beschriebene Drogenzusammensetzung enthält neben Eichenrinde und Süßholzwurzel Wurzel von *Bupleurum falcatum, Heracleum dulce, Ledebouriella seseloides,* Wurzel von *Platycodon grandiflorum,* Rhizom von *Cnidium officieale, Poria* cocos, *Schizonepeta tenuifolia,* und *Zingiberis siccatum rhizoma.*

### 2.1. Drogen und Drogenzubereitung

Als Ausgangsmaterial wurde *Quercus cortex* (Mischung aus Q. *robur* und *Q. petraea,* Herkunft Nr. Qrp4) und *Liquiritiae radix* (Herkunft Nr. GG 12) der Firma Vitaplant eingesetzt. Beide Drogen entsprechen der vorgeschriebenen Qualität des europäischen Arzneibuches.

Die Drogen wurden in einer Zentrifugalmühle pulverisiert (Siebgröße 0,75) und anschließend gemischt.

### 2.2. Extraktion

### 2.2.1. Wässrig-ethanolischer Extrakt

Der Extrakt wurde aus einer Drogenmischung hergestellt, die sich aus einem Teil *Quercus cortex* und zwei Teilen *Liquiritiae radix* (Wurzel von *Glycyrrhiza glabra*), entsprechend dem obigen Beispiel 1, zusammensetzt.

Es wurde 5,0 g pulverisierte Drogenmischung eingewogen und mit 100 ml 70 % wässrigem Ethanol für 10 min im Ultraschallbad extrahiert. Nach einer Filtration wurde der Rückstand noch einmal mit 50 ml des gleichen Lösungsmittels unter gleichen Bedingungen nachextrahiert. Der Extrakt wurde am Vakuum auf ca. 80 ml eingeengt. Anschließend wurde das Volumen mit 70 % Ethanol auf 100 ml eingestellt.

### 2.2.2. Wässriger Extrakt

Ein Teil pulverisierte *Quercus cortex* und ein Teil pulverisierte *Liquiritiae radix* wurden gemischt. 5,0 g dieser Mischung wurden zweimal mit 50 ml Wasser (bidest. Qualität) im Heizbad zum Sieden erhitzt, anschließend filtriert und das Volumen des Extraktes auf 100 ml mit Wasser ergänzt.

Beide Extrakte wurden über Nacht bei 5 °C gelagert. Es zeigte sich, dass im wässrigen Extrakt nach der Abkühlung einige Stoffe ausfielen, im Gegensatz zum ethanolischen Extrakt, der keine Trübung aufwies.

### 2.3. Analyse

Die beiden Extrakte wurden mit Hilfe einer hochauflösenden Dünnschichtchromatographie untersucht. Als stationäre Phase für die Dünnschichtchromatographie wurden Kieselgelplatten 60 F₂₅₄ der Firma Merck eingesetzt. Als Laufmittel wurde 1-Butanol:Ammoniak (25 %): Ethanol (50:20:10) eingesetzt. Für die Derivatisierung wurde Anisaldehydreagenz, 5 min bei 105 °C, verwendet. Die Detektion und Dokumentation erfolgte bei Tageslicht. Als Referenzsubstanzen wurden +-Catechin, α-Glycyorrhetinsäure und Glycyrrhicinsäure, jeweils 1 mg/ml Methanol, eingesetzt. Die Auftragsmenge für die Extrakte und die Referenzsubstanzen betrug jeweils 8 µl.

Die Lösungen wurden mit einem Linomat-Auftragegerät (Firma Camag) auf die Dünnschichtchromatographieplatten aufgetragen. Die Derivatisierung erfolgte nach dem Eintauchen für zwei Sekunden in die Anisaldehylösung auf einer Dünnschichtchromatographie-Wärmeplatte. Die Dünnschichtchromatographieplatten wurden unter Vis mit einem Videostore-System (Firma Camag) ausgewertet und dokumentiert.

### 2.4. Resultate und Diskussion

Obwohl mit der hier eingesetzten Dünnschichtchromatographiemethode nur ein bestimmtes Spektrum von Inhaltsstoffen sichtbar gemacht werden kann, da es sich hierbei um eine für glykosidische Verbindungen entwickeltes System handelt, ließen sich dennoch deutliche Unterschiede in der Inhaltsstoffzusammensetzung der beiden Extrakttypen erkennen. Im Ethanolextrakt waren vermehrt Stoffe aus dem lipophilen Bereich extrahiert worden. Diese Verbindungen waren im oberen Bereich des Chromatogramms zu erkennen. Beim Wasserextrakt fehlten die Banden in dieser Zone. Weiterhin war auffällig, dass α-Glycyrrhetinsäure ausschließlich im ethanolischen Extrakt nachzuweisen war. Ob Glycyrrhizin in beiden Extrakttypen vorhanden ist, ließ sich bei diesem Vergleichsversuch nicht eindeutig erkennen. Allgemein ließen sich im ethanolischen Extrakt generell höhere Gehalte an Inhaltsstoffen nachweisen als im Wasserextrakt.

Im Ergebnis wurde gezeigt, dass durch die ethanolische Extraktion einer Mischung aus *Quercus cortex* und *Liquiritiae radix* ein anderes Inhaltsstoffspektrum gewonnen wird als durch wässrige Extraktion. Insbesondere enthält der erfindungsgemäße ethanolische Extrakt neben den rein wasserlöslichen Inhaltsstoffen im Gegensatz zum wässrigen Extrakt zusätzlich lipophile Substanzen. Von besonderer Wirkung scheint die ausschließlich im erfindungsgemäßen ethanolischen Extrakt nachzuweisende Glycyrrhetinsäure zu sein, die pharmakologisch als wesentlicher Bestandteil der Süßholzwurzel angesehen wird. Weiterhin ist davon auszugehen, dass Substanzen ähnlicher Struktur ebenfalls zu den Wirkstoffen zählen und entsprechend wie Glycyrrhetinsäure mit den erfindungsgemäß eingesetzten lipophilen Extraktionsmitteln aus dem Drogenmaterial gewonnen werden. Im Ergebnis wird also durch die erfindungsgemäße ethanolische Extraktion eine andere Wirkstoffzusammensetzung als mit wässriger Extraktion erreicht, so dass dem ethanolischen Extrakt ein anderes Wirkspektrum als dem wässrigen Extrakt zuzuschreiben ist.

## Patentansprüche

1. Pharmazeutisches Kombinationspräparat, enthaltend einen Extrakt aus Süßholzwurzel (Glycyrrhiza glabra) und Eichenrinde (Quercus spec.).

2. Pharmazeutisches Kombinationspräparat nach Anspruch 1, **dadurch gekennzeichnet, daß** der Extrakt auf einem Auszug mit einem organischen Lösungsmittel, insbesondere einem mit Wasser mischbaren Lösungsmittel, vorzugsweise auf einem Auszug mit einem Alkohol/Wasser-Gemisch, insbesondere Ethanol/Wasser-Gemisch, beruht.

3. Pharmazeutisches Kombinationspräparat nach Anspruch 2, **dadurch gekennzeichnet, daß** das Auszugsmittel für den Auszug aus etwa 20 bis etwa 80 Vol.% Ethanol, vorzugsweise etwa 40 bis etwa 70 Vol.% Ethanol, und etwa 20 bis etwa 80 Vol.% Wasser, vorzugsweise etwa 30 bis etwa 60 Vol.% Wasser, besteht.

4. Pharmazeutisches Kombinationspräparat nach einem der vorhergehenden Ansprüche, **dadurch gekennzeichnet, daß** es ein Extrakt aus etwa 1 bis etwa 5 Teile Süßholzwurzel, vorzugsweise etwa 2 Teile Süßholzwurzel, und etwa 1 bis etwa 3 Teile Eichenrinde, vorzugsweise etwa 1 Teil Eichenrinde, ist.

5. Pharmazeutisches Kombinationspräparat nach einem der vorhergehenden Ansprüche, **dadurch gekennzeichnet, daß** es ein Extrakt aus einer Mischung aus Süßholzwurzel und Eichenrinde ist, insbesondere aus getrockneter und/oder geschnittener Süßholzwurzel und Eichenrinde.

6. Pharmazeutisches Kombinationspräparat nach einem der vorhergehenden Ansprüche, **dadurch gekennzeichnet, daß** es als flüssiger Auszug vorliegt.

7. Pharmazeutisches Kombinationspräparat nach einem der vorhergehenden Ansprüche, **dadurch gekennzeichnet, daß** es mindestens teilweise in getrockneter Form, insbesondere als Paste oder Pulver, vorliegt.

8. Pharmazeutisches Kombinationspräparat nach einem der vorhergehenden Ansprüche, **dadurch gekennzeichnet, daß** es als Mischung mit einem als Nahrungsmittel, Genußmittel und/oder Körperpflegemittel dienenden Träger vorliegt.

9. Pharmazeutisches Kombinationspräparat nach einem der vorhergehenden Ansprüche, **dadurch gekennzeichnet, daß** es zur Behandlung von viralen Erkrankungen vorgesehen ist.

10. Pharmazeutisches Kombinationspräparat nach Anspruch 9, **dadurch gekennzeichnet, daß** die Symptome der viralen Erkrankung im Lippen- und/oder Rachenraum vorhanden sind.

11. Pharmazeutisches Kombinationspräparat nach Anspruch 9 oder Anspruch 10, **dadurch gekennzeichnet, daß** die virale Erkrankung eine Herpes-Erkrankung, insbesondere eine Herpes labialis-Erkrankung ist.

12. Verwendung von Süßholzwurzel (Glycyrrhiza glabra) und Eichenrinde (Quercus spec.) zur Herstellung einer pharmazeutischen Zusammensetzung.

13. Verwendung von Süßholzwurzel (Glycyrrhiza glabra) und Eichenrinde (Quercus spec.) zur Herstellung einer pharmazeutischen Zusammensetzung zur Behandlung von viralen Erkrankungen.
